# FASCICULE DE BREVET EUROPEEN

(11) **EP 1 635 710 B1**
(45) Date de publication et mention de la délivrance du brevet: **13.08.2008**
(21) Numéro de dépôt: 04767311.6
(22) Date de dépôt: 10.06.2004
(51) Int. Cl.: A61B 10/00

(54) **TROCART DE PONCTION DE MOELLE OSSEUSE**
KNOCHENMARKSASPIRATIONSTROKAR
BONE MARROW ASPIRATION TROCAR

(30) Priorité: 12.06.2003 FR 0307064
(43) Date de publication de la demande: 22.03.2006
(73) Titulaire: Verra, Olivier, 84000 Avignon (FR); Verra, Florence HF, 84000 Avignon (FR); Verra, Raphael HM, 84270 Vedène (FR); Slama, Borhane, 84000 Avignon (FR); Zerazhi, Hacène, 13570 Barbentane (FR)
(72) Inventeur: SLAMA, Borhane, F-84000 Avignon (FR); ZERAZHI, Hacène, F-13570 Barbentane (FR); VERRA, Yvan DI, . (FR)
(74) Mandataire: Dall'Olio, Giancarlo
(86) Numéro de dépôt international: PCT/FR2004/001446
(87) Numéro de publication internationale: WO 2004/110281

(56) Documents cités:
- GB-A- 2 130 890
- US-A- 5 012 818
- US-A- 5 122 114

## Description

La présente invention concerne un dispositif (trocart) de ponction de moelle osseuse.

Dans le domaine médical, il est parfois nécessaire de faire une ponction de la moelle osseuse, à des fins d'analyse quantitative et/ou qualitative, ou à d'autres fins : greffes etc... Les ponctions se font en général à l'intérieur de trois os du corps humain : l'os iliaque antérieur ou l'os iliaque postérieur ou le sternum.

Pour procéder à cette ponction, et après préparation du patient, asepsie en particulier, il faut traverser la peau (l'épiderme et derme) puis le périoste puis la partie externe de l'os appelée « table externe » avant d'atteindre la moelle. Il faut prendre soin de ne pas traverser l'os qui se trouve au-delà de la moelle qui s'appelle « table interne » car d'autres organes se trouvent derrière ledit os.

L'os ainsi que la moelle ayant des épaisseurs variables suivant les individus et suivant le point exact du prélèvement, cet acte demande un longue pratique, du doigté, un savoir-faire certain du praticien qui doit et un instrument appelé « trocart de ponction de moelle ».

### ARRIERE PLAN DE L'INVENTION

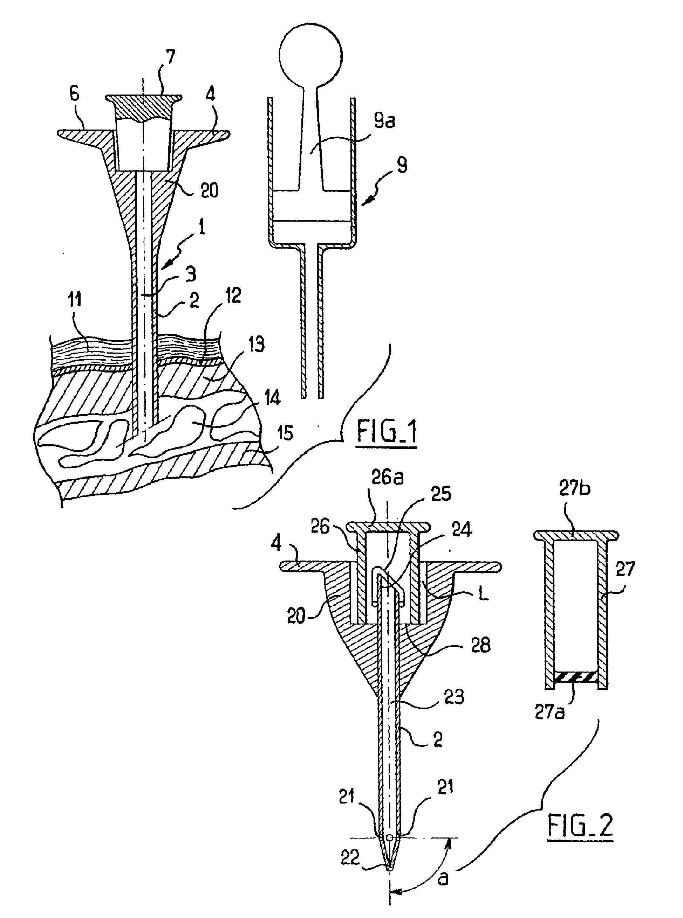

On connaît des trocarts à usage multiple, stérilisable (métallique) ou sous forme jetable à usage unique (plastique avec une aiguille métallique). La figure 1 illustre une réalisation de cet instrument connu.

A cette figure, un trocart 1 de perforation de l'os, comprend un corps creux formant, à une extrémité, un tube creux métallique 2 en forme d'aiguille creuse et à l'autre extrémité un corps de préhension 20 conique avec des oreilles 4 et une surface supérieure 6.

Une tige d'obturation 3 du trocart doit être introduite dans l'aiguille 2 avant toute poussée de pénétration. Cette tige possède un embout qui possède une surface de poussée 7 au centre de celle du corps 20. Cette tige interdit l'introduction dans l'aiguille de matières (peau, débris d'os, etc...) susceptibles de boucher le trocart 1 et qui pourraient empêcher l'aspiration de la moelle qui se fera au moyen d'une seringue 9 d'aspiration manuelle à piston 9a, (voir egalement GB2130890).

Cette seringue d'aspiration 9 avec piston actionné manuellement permet au praticien d'aspirer la moelle osseuse 4 après avoir enlevé la tige d'obturation 3, en la remplaçant par ladite seringue d'aspiration.

Les gestes du praticien consistent à introduire la tige d'obturation 3 dans le trocart 1, puis à choisir l'endroit où il va pénétrer dans l'os, à présenter l'aiguille 2 du trocart et à pousser pour traverser la peau 11, puis le périoste 12, puis la table externe de l'os 13 jusqu'aux poches de moelle 14 sans traverser la partie arrière 15 qui s'appelle la table interne.

Lorsque le praticien pense que l'extrémité de l'aiguille du trocart se trouve dans la zone où l'aspiration est possible, il stoppe la poussée, il enlève la tige d'obturation 3, il met en place la seringue 9 et manuellement, il tente d'aspirer la moelle en tirant sur le piston 9a.

Quelle que soit l'expérience du praticien, la recherche de la zone d'aspiration reste délicate et surtout aléatoire. Il est très fréquent que cette traction du piston ne produise aucun effet.

En cas d'échec, le praticien doit répéter et ce, souvent plusieurs fois, toute la série de ces opérations, c'est-à-dire enlever la seringue, puis remettre la tige d'obturation, puis pousser sur le trocart pour aller un peu plus profondément, stopper la poussée, enlever la tige d'obturation, puis remettre la seringue, tenter de nouveau une aspiration, et ainsi de suite. Ces opérations successives entraînent des conséquences qui ne sont pas neutres :
- majoration du risque infectieux par l'allongement de l'acte, et par mise à l'air successives et prolongées des composants
- perte du temps précieux du praticien et de son assistant, certains actes peuvent se prolonger jusqu'à vingt minutes... .
- douleur du patient qui se prolonge pendant ce temps...
- difficulté, parfois, pour réintroduire la tige d'obturation dans le trocart. Il faut parfois tout recommencer, perforation incluse....
- douleur surajoutée du patient lors de cette réintroduction ..

Et dans les cas extrêmes (à force de pousser l'aiguille en avant) risque de perforation de la table interne ce qui peut provoquer la perforation d'organes sous-jacents, cela s'étant déjà produit.

### OBJET DE L'INVENTION

Les inventeurs ont conçu un nouveau trocart de ponction de moelle osseuse qui supprime la plupart des inconvénients ci-dessus : tout en améliorant le confort du patient, il rend le prélèvement plus sûr et diminue la durée de l'acte du praticien, tout en le sécurisant.

### BREVE DESCRIPTION DE L'INVENTION

La présente invention a pour objet un dispositif de ponction de moelle osseuse liquide comportant un élément en forme d'aiguille creuse dont une extrémité de préhension possède une poignée et dont une extrémité opposée est une extrémité d'insertion, dans lequel l'extrémité d'insertion est fermée au bout et porte des trous d'aspiration latéraux.

Le trocart est fermé et son extrémité d'insertion pourra être taillée en pointe ou en biseau ou en toute autre forme adaptée à l'endroit du prélèvement sur le patient, dont l'action devient alors une action d'écartement et non plus une découpe des matières, évitant le détachement de particules pouvant boucher le canal de prélèvement.

De manière préférée, l'aiguille creuse forme à son extrémité de préhension une tige perforante creuse débouchant dans un logement de la poignée, destinée à pénétrer le diaphragme étanche d'une enceinte sous vide. Il s'agit là d'une capsule formant à la fois source de vide et d'aspiration et récipient de recueil du liquide aspiré.

D'autres caractéristiques et avantages de l'invention ressortiront de la description d'un exemple de réalisation donnée ci-après.

### BREVE DESCRIPTION DES DESSINS

Il sera fait référence aux dessins annexés parmi lesquels :
- la figure 1 illustre un dispositif conforme à l'état de la technique,
- la figure 2 est un schéma d'une réalisation du dispositif de l'invention.

### DESCRIPTION DETAILLEE DE L'INVENTION

Le trocart de l'invention représenté à la figure 2 est une aiguille creuse et fermée à son extrémité (22) d'insertion qui pourra être taillée en pointe ou en biseau , dont l'action devient une action d'écartement et n'est plus une découpe des matières, évitant ainsi le bouchage par les particules de matière qui ne sont plus découpées. Cette extrémité fermée permet surtout la suppression de la tige d'obturation 3 (Figure 1) qui n'existe plus dans le nouveau trocart. D'où une simplification de l'instrument et une simplification de l'acte du praticien qui n'a plus à enlever la tige d'obturation (et éventuellement la remettre, pour l'enlever de nouveau, comme décrit ci-dessus) entraînant les risques décrits (rappel : risque infectieux, perte de temps, douleur et douleur surajoutée).

Le trocart selon l'invention est muni d'un ou plusieurs trous d'aspiration latéraux 21 situés au-dessus de l'extrémité fermée 22. Ces trous n'étant pas débouchant directement dans l'axe de la poussée, mais inclinés par rapport à cet axe d'un angle a au moins égal à 90° , ne laissent pas pénétrer de micro-particules de matière. Le diamètre de ces trous 21 a été déterminé pour que chacun soit préférentiellement plus petit que le diamètre intérieur de l'aiguille 23 du nouveau trocart. Ainsi, même dans le cas très improbable où des particules passeraient par l'un des trous, il leur serait impossible de boucher le conduit intérieur de l'aiguille du trocart (23) qui est d'un diamètre plus grand que les trous d'entrée (21).

Par ailleurs, le trocart selon l'invention est doté à son autre extrémité d'une tige perforante creuse (24) coiffée par une capote d'asepsie 25.

Autour de la tige perforante creuse 24, un corps ou poignée 20 de préhension à oreilles horizontales 4 présente un logement L avec une surface de fond formant une surface d'appui 28 pour un capuchon amovible 26 qui peut se loger dans le logement L afin de le boucher et offrir au praticien une cloison 26a de poussée supérieure pour appuyer fortement sur le trocart et procéder à la perforation de la table externe de l'os. On retire le bouchon pour mettre en place une capsule 27 qui entre dans le logement L et qui s'appuie sur la surface 28, un diaphragme 27a ayant été perforé lors de cette mise en place par la tige 24. La capsule 27 forme une enceinte sous vide partiel qui se communique au trocart jusqu'aux orifices d'aspiration 21. Elle possède une cloison supérieure 27b de poussée comme le capuchon 26.

Grâce à cette invention, l'acte du praticien est considérablement changé, optimisé et sécurisé, comme le démontre sa description :

Le praticien introduit le trocart dans la table externe de l'os 13 jusqu'à ce que les trous latéraux d'aspiration 21 ont dépassé la surface externe. Il enlève alors le capuchon 26 et le remplace par la capsule sous vide transparente 27 dans le logement L.

Il prolonge alors son mouvement de pénétration.

Dès que les trous d'aspiration latéraux 21 seront au premier contact de la moelle, elle sera instantanément et automatiquement aspirée par le vide régnant dans le trocart et la capsule 27 où la moelle apparaît immédiatement à la vue du praticien qui stoppe son mouvement et doit désormais uniquement surveiller le volume souhaité du prélèvement dans la capsule 27.

En interchangeant tout simplement la capsule pleine par une ou plusieurs autres capsules, le praticien peut faire d'autres prélèvements rapidement, dans des conditions optimales d'asepsie, avec une amélioration du confort du patient puisque l'intervention est très rapide. De plus, à l'intérieur de la capsule, le prélèvement est isolé de l'atmosphère ambiante ce qui sécurise la qualité du prélèvement.

Enfin, notons que le remplacement de plusieurs capsules 27 en un temps très court permet de faire des « analyses instantanées en continu » dans des conditions optimales d'asepsie.

Les avantages de l'invention sont nombreux. On citera
- l'optimisation du prélèvement médullaire,
- la diminution du risque infectieux par la ponction instantanée, sans mise à l'air libre, des prélèvements,
- la suppression de la réintroduction de la tige d'obstruction au coeur de la moelle après être passée dans l'air ambiant lors des tentatives successives infructueuses de ponction,
- un gain de temps précieux pour le praticien, son assistant et les autres patients qui attendent,
- un raccourcissement de la durée de la douleur du patient grâce à la rapidité de l'acte et la suppression de toute douleur supplémentaire grâce à la suppression des manipulations lors des phases de réintroduction de la tige d'obturation (3),
- la mise en oeuvre de plusieurs modèles de formes de capsules afin que les prélèvement soient immédiatement conditionnés de manière adaptée aux différentes techniques d'examens (ce qui n'est pas possible avec les seringues à piston manuel de l'ancien trocart). Ainsi, chaque capsule peut avoir une section particulière ou un signe ou une couleur qui est le signe de sa destination.

Bien entendu, on aura prévu des trocarts dont l'aiguille est de longueur adaptée à l'os dans lequel il faut prélever ; ce sera une petite aiguille de prélèvement s'il s'agit d'un os plat comme le sternum ou une aiguille plus longue lorsqu'il conviendra d'opérer sur par exemple l'os illiaque.

## Revendications

1. Dispositif de ponction de moelle osseuse liquide comportant un élément en forme d'aiguille creuse avec à une extrémité un corps de préhension avec une poignée (4) et une extrémité d'insertion (22), fermée au bout et portent des trous d'aspiration latéraux (21), **caractérisé** et en ce que l'aiguille creuse forme à son extrémité de préhension une tige perforante creuse (24) saillant dans un logement (L) du corps de préhension (20) destinée à perforer le diaphragme étanche (27) d'une enceinte (26) sous vide.

2. Dispositif selon l'une quelconque des revendications 1, **caractérisé en ce que** le diamètre des trous latéraux d'aspiration (21) est inférieur au diamètre intérieur de ladite aiguille.

3. Dispositif selon l'une quelconque des revendications précédentes, **caractérisé en ce que** l'axe de chaque trou (21) d'aspiration forme avec l'axe de pénétration de l'aiguille (23) un angle d'au moins 90°.

4. Dispositif selon la revendication 1, **caractérisé en ce qu'**il comprend un capuchon amovible (25) de la tige perforante de l'aiguille, adapté au logement (L) du corps de préhension (20) pour présenter au praticien une surface de poussée au droit du logement.

5. Dispositif selon la revendication 1, **caractérisé en ce que** l'enceinte à vide est d'une forme extérieure adaptée à pénétrer dans le logement (1) du corps de préhension (20) pour présenter au praticien une surface de poussée au droit de ce logement (L).

6. Dispositif selon la revendication 1, **caractérisé en ce qu'**il comporte un jeu d'enceintes à vide sous forme de capsules (26) différentes les unes des autres en fonction de leur destination.

## Claims

1. Device for aspirating liquid bone marrow, comprising a hollow needle-shaped element with, at one end, a holding body with a hand grip (4) and an end for insertion (22) closed at the tip and carrying lateral aspiration holes (21), **characterised in that** the hollow needle forms, at its holding end, a hollow piercing rod (24) protruding into a seat (L) of the holding body (20) for piercing the sealed diaphragm (27) of a chamber (26) under vacuum.

2. Device according to claim 1, **characterised in that** the diameter of the lateral aspiration holes (21) is smaller than the inner diameter of said needle.

3. Device according to any one of the preceding claims, **characterised in that** the axis of each aspiration hole (21) forms an angle of at least 90 ° with the axis of penetration of the needle (23).

4. Device according to claim 1, **characterised in that** it comprises a detachable cover (25) for the piercing rod of the needle, suitable for the seat (L) of the holding body (20) so as to provide the practitioner with a surface to push to the right of the seat.

5. Device according to claim 1, **characterised in that** the empty chamber has an outer shape which is suitable for penetrating into the seat (L) of the holding body (20) so as to provide the practitioner with a surface to push on to the right of said seat (L).

6. Device according to claim 1, **characterised in that** it comprises a set of empty chambers in the form of caps (26) which are different from one other as a function of their use.

## Patentansprüche

1. Vorrichtung zur Aspiration von Knochenmarksflüssigkeit, die ein Element in Form einer Hohlnadel mit einem Greifkörper mit einem Griff (4) an einem Ende und einem Ende zur Einführung (22), das am Ende geschlossen ist und das seitliche Absauglöcher (21) trägt, umfasst, **dadurch gekennzeichnet, dass** die Hohlnadel an ihrem Greifende einen hohlen perforierende Stiel (24) bildet, welcher in eine Aufnahme (L) des Greifkörpers (20) hineinragt, dazu bestimmt, die dichte Blende (27) eines unter Vakuum stehenden Innenraums (26) zu perforieren.

2. Vorrichtung gemäß Anspruch 1, **dadurch gekennzeichnet, dass** der Durchmesser der seitlichen Absauglöcher (21) geringer als der innere Durchmesser der Nadel ist.

3. Vorrichtung gemäß einem der vorhergehenden Ansprüche, **dadurch gekennzeichnet, dass** die Achse jedes Absauglochs (21) mit der Achse des Eindringens der Nadel (23) einen Winkel von mindestens 90° bildet.

4. Vorrichtung gemäß Anspruch 1, **dadurch gekennzeichnet, dass** sie eine an die Aufnahme (L) des Greifkörpers (20) angepasste, abnehmbare Verschlusskappe (25) des perforierenden Stiels der Nadel umfasst, um dem Praktiker eine Druckoberfläche zur Geraden der Aufnahme vorzustellen.

5. Vorrichtung gemäß Anspruch 1, **dadurch gekennzeichnet, dass** der unter Vakuum stehende Innenraum eine äußere Form aufweist, die zum Eindringen in die Aufnahme (1) des Greifkörpers (20) angepasst ist, um dem Praktiker eine Druckoberfläche zur Geraden dieser Aufnahme (L) vorzustellen.

6. Vorrichtung gemäß Anspruch 1, **dadurch gekennzeichnet, dass** sie einen Satz von unter Vakuum stehenden Innenräumen in Form von Kapseln (26), die abhängig von ihrer Bestimmung eine von der anderen unterschiedlich sind, umfasst.
